# EUROPEAN PATENT SPECIFICATION

(11) **EP 3 866 818 B1**
(45) Date of publication and mention of the grant of the patent: **23.07.2025**
(21) Application number: 19827796.4
(22) Date of filing: 18.10.2019
(51) Int. Cl.: A61K 35/60, A61P 17/02

(54) **USE OF FISH EGG CELLULAR EXTRACTT FOR WOUND HEALING**
VERWENDUNG VON FISCH EI ZELLEXTRAKT ZUR WUNDHEILUNG
UTILISATION D'EXTRAIT CELLULAIRE D'OEUF DE POISSON POUR TRAITER LES BLESSURES

(30) Priority: 19.10.2018 US 201862747797 P
(43) Date of publication of application: 25.08.2021
(73) Proprietor: Regenics AS, 0485 Oslo (NO)
(72) Inventor: LUND, Henrik, 0485 Oslo (NO); KALSTAD LØNNE, Gry, 0485 Oslo (NO); CLEMM, Christian, 0485 Oslo (NO)
(74) Representative: Algemeen Octrooi- en Merkenbureau B.V.
(86) International application number: PCT/IB2019/001130
(87) International publication number: WO 2020/079484

(56) References cited:
- WO-A1-01/89540
- WO-A2-2008/020329
- WO-A2-2014/091312
- CN-A- 103 251 652
- P. BANNON ET AL: "Diabetes induces stable intrinsic changes to myeloid cells that contribute to chronic inflammation during wound healing in mice", DISEASE MODELS & MECHANISMS, vol. 6, no. 6, 18 September 2013 (2013-09-18), GB, pages 1434 - 1447, XP055666209, ISSN: 1754-8403, DOI: 10.1242/dmm.012237

## Description

### FIELD OF THE INVENTION

The present invention provides compositions and methods for the improved healing of chronic wounds.

### BACKGROUND OF THE INVENTION

Chronic wounds represent a significant burden to patients, health care professionals, and the US health care system, affecting 5.7 million patients and costing an estimated 20 billion dollars annually.

Chronic wounds are rarely seen in individuals who are otherwise healthy. In fact, chronic wound patients frequently suffer from "highly branded" diseases such as diabetes and obesity. Chronic wounds are those that have failed to proceed through an orderly and timely reparative process to produce anatomic and functional integrity of the injured site. Often disguised as a comorbid condition, chronic wounds represent a silent epidemic that affects a large fraction of the world population and poses major and gathering threat to the public health and economy of the United States. In developed countries, it has been estimated that 1 to 2% of the population will experience a chronic wound during their lifetime. In the United States alone, chronic wounds affect 6.5 million patients. In the Scandinavian countries, the associated costs account for 2-4% of the total health care expenses.

The burden of treating chronic wounds is growing rapidly due to increasing health care costs, an aging population and, in the United States and beyond, a sharp rise in the incidence of diabetes and obesity worldwide. It is claimed that an excess of US$25 billion is spent annually on treatment of chronic wounds. To that add the rapidly expanding need for wound care of our veterans, and the need to prioritize wound care and research would appear to be compelling. At present, over 1000 outpatient wound centers are in operation in the United States, not including all the wound care rendered by clinicians in their offices, by inpatient acute care hospitals, long term facilities and nursing homes. According to a new report by Global Industry Analysts, the annual wound care products market would reach $15.3 billion by 2010. The United States represents the world's largest and the fastest growing market. The amount of money spent on wound care, the loss of productivity for afflicted individuals and the families that care for them and their diminished quality of life come at great cost to our society.

What is needed in the art are new and effective treatments for chronic wounds.

### SUMMARY OF THE INVENTION

The present disclosure provides compositions and methods for the improved healing of chronic wounds.

In some embodiments, the present invention provides for an egg cellular extract for use in promopting, accelerating, or improving healing of chronic wounds in a diabetic subject, wherein said egg cellular extract is fish egg cellular extract. In an embodiment said chronic wound is a diabetic ulcer. In an embodiment the use increases the ratio of alternatively activated macrophages: classically activated macrophages at the site of the wound upon application of said egg cellular extract. In an embodiment the use increases the expression and/or release by macrophages of one or more cell mediators selected from the group consisting of IL-1 RA, IL-6, IL-10 and VEGF. In an embodiment the use decreases the expression and/or release by macrophages of one or more cell mediators selected from the group consisting of IL-12, TNF-α, MCP-1, and IL-8. In an embodiment the use increases expression of AMAC-1 by macrophages. In an embodiment said fish egg cellular extract is from an unfertilized egg. In an embodiment said cellular extract is obtained by heat treating the cellular extractby heating the extract to greater than 80C, 90C, 95C or 100C. In an embodiment said heat treatment is from about 1 minute to about 30 minutes. In an embodiment said egg cellular extract is provided in a cream, gel, emulsion, ointment, spray, powder or lotion. In an embodiment said egg cellular extract is a cytoplasmic extract. In an embodiment the use produces the effect as defined in claims 3-6 at the site of a wound in a subject.

### BRIEF DESCRIPTION OF THE FIGURES

FIG. 1 is a graph showing reduction of selected inflammatory markers in human fibroblasts pretreated with salmon roe extract (0.5 % w/v) vs. control (no pre-treatment).
FIG. 2 is a graph showing reduction of IL-1β in human macrophages pretreated with salmon roe extract (0.5 % w/v) vs. control (no pre-treatment).

### DEFINITIONS

"Anti-infective agents" include, but are not limited to benzylpenicillin, penicillin, penicillin G, 6-phenyl acetyl penicillin, penicllin V, micronomicin, clavulanate, oxacillin, dequalinium, cloxacillin, sulbenicillin, ampicillin, cilleral, and principen and combinations thereof.

"Anti-inflammatory" means a substance that reduces inflammation. Many analgesics remedy pain by reducing inflammation. Many steroids - specifically glucocorticoids - reduce inflammation by binding to cortisol receptors. Non-steroidal anti-inflammatory drugs (NSAIDs) alleviate pain by counteracting the cyclooxygenase (COX) enzyme. On its own COX enzyme synthesizes prostaglandins, creating inflammation. Many herbs have anti-inflammatory qualities, including but not limited to hyssop and willow bark (the latter of which contains salicylic acid, the active ingredient in aspirin), as well as birch, licorice, wild yam and ginseng.

"Antioxidants" means any of a variety of substances that prevent or slow the breakdown of another substance by oxygen. Synthetic and natural antioxidants are used to slow the deterioration of gasoline and rubber, and such antioxidants as vitamin C (ascorbic acid), butylated hydroxytoluene (BHT), and butylated hydroxyanisole (BHA) are typically added to foods to prevent them from becoming rancid or from discoloring. Nutrients such as beta-carotene (a vitamin A precursor), vitellogenin, vitamin C, vitamin E, and selenium have been found to act as antioxidants. They act by scavenging free radicals, molecules with one or more unpaired electrons, which rapidly react with other molecules, starting chain reactions in a process called oxidation. Free radicals are a normal product of metabolism; the body produces its own antioxidants (e.g., the enzyme superoxide dismutase) to keep them in balance. However, stress, aging, and environmental sources such as polluted air and cigarette smoke can add to the number of free radicals in the body, creating an imbalance. The highly reactive free radicals can damage healthy DNA and have been linked to changes that accompany aging (such as age-related macular degeneration, a leading cause of blindness in older people) and with disease processes that lead to cancer, heart disease, and stroke.

An "antiseptic" is a substance that kills or prevents the growth and reproduction of various microorganisms, including bacteria, fungi, protozoa, and viruses on the external surfaces of the body. The objective of antiseptics is to reduce the possibility of sepsis, infection or putrefaction by germs. Antibacterials have the same objective but only act against bacteria. Antibiotics perform a similar function, preventing the growth or reproduction of bacteria within the body. Antiseptics include, but are not limited to, alcohol, iodine, hydrogen peroxide, and boric acid. There is great variation in the ability of antiseptics to destroy microorganisms and in their effect on living tissue. For example, mercuric chloride is a powerful antiseptic, but it irritates delicate tissue. In contrast, silver nitrate kills fewer germs but can be used on the delicate tissues of the eyes and throat. There is also a great difference in the time required for different antiseptics to work. Iodine, one of the fastest-working antiseptics, kills bacteria within 30 sec. Other antiseptics have slower, more residual action. Since so much variability exists, systems have been devised for measuring the action of an antiseptic against certain standards. The bacteriostatic action of an antiseptic compared to that of phenol (under the same conditions and against the same microorganism) is known as its phenol coefficient.

A "gel" is a semisolid material formed from a colloidal solution. By weight, gels are mostly liquid, yet they behave like solids. An example is gelatin.

"Keratin" is any of a variety of fibrous protein molecules that serve as structural units for various living tissues. The keratins are the major protein components of hair, wool, nails, horn, hoofs, and the quills of feathers. These proteins generally contain large quantities of the sulfur-containing amino acids, particularly cysteine. The helical keratin molecules twist around each other to form elongated strands called intermediate filaments. The formation of a disulfide bridge between the sulfur atoms on two cysteines on separate polypeptide chains of keratin allows for the cross-linkage of these chains and results in a fairly rigid aggregate.

"Immunomodulator" means any of a variety of substance that influences the immune system. Examples include, but are not limited to, cytokines, Interleukin-2, immunostimulants, and immunosuppressors.

The term "natural product" means any of a variety of organic chemical moieties whose molecular arrangement is derived from enzymatic transformations in a living organism excluding amino acids, proteins, polypeptides, nucleic acids and sequences, and saturated fatty acids. Examples include, but are not limited to lipids (i.e., that are not saturated fatty acids), carbohydrates/saccharides and polysaccharides, the steroids and their derivatives, the terpenes and their derivatives, vitamins, carotenoids, and natural medicines such as taxol, etc. The term "synthetic natural product" is a natural product not obtained from its natural source.

"Cell" means the smallest structural unit of living matter capable of functioning autonomously, consisting of one or more nuclei, cytoplasm, and various organelles, all surrounded by a semipermeable membrane. Cells include all somatic cells obtained or derived from a living or deceased animal body at any stage of development as well as germ cells, including sperm and eggs (animal reproductive body consisting of an ovum or embryo together with nutritive and protective envelopes). Included are both general categories of cells: prokaryotes and eukaryotes. The cells contemplated for use in this disclosure include all types of cells from all organisms in all kingdoms: plans, animals, protists, fungi, archaebacteria and eubacteria. Stem cells are cells capable, by successive divisions, of producing specialized cells on many different levels. For example, hematopoietic stem cells produce both red blood cells and white blood cells. From conception until death, humans contain stem cells, but in adults their power to differentiate is reduced.

As used herein, the term "differentiation" related to cells means the process by which cells becomes structurally and functionally specialized, which is a progressive restriction of the developmental potential and increasing specialization of function which takes place during the development of the embryo and leads to the formation of specialized cells, tissues, and organs.

The term "dedifferentiation" related to cells means the reverse process of differentiation, where cells become less structurally and functionally specialized, which increases the developmental potential of the cell.

"Differentiable" means the ability of a cell to differentiate into a desired cell type. As used herein, the term "differentiates" means specialization (differentiation) or return to a more primitive cell type; dedifferentiation).

An "extract" as used in the context of "cell extract" and "egg extract" in this disclosure means a preparation of any type of cell as defined above obtained by chemical or mechanical action, as by pressure, distillation, evaporation etc. Extracts can include all or any single component or combination of components of the cells, including concentrated preparations of the active components. Such components of the extracts include but are not limited to RNA, DNA, micro RNA, lipids, free amino acids, all amino acid base structures including peptides and proteins, carbohydrates, minerals or combinations thereof. Extracts contemplated by this disclosure include but are not limited to extracts of fish eggs, urchin eggs, frog eggs, adult stem cells, plant seeds and plant stem cells. Extracts contemplated by the invention are fish egg cellular extracts.

The term "manage" when used in connection with a disease or condition means to provide beneficial effects to a subject being administered with a prophylactic or therapeutic agent, which does not result in a cure of the disease. In certain embodiments, a subject is administered with one or more prophylactic or therapeutic agents to manage a disease so as to prevent the progression or worsening of the disease.

As used herein, the terms "prevent" and "preventing" include the prevention of the recurrence, spread or onset. It is not intended that the present invention be limited to complete prevention. In some embodiments, the onset is delayed, or the severity of the disease is reduced.

As used herein, the terms "treat" and "treating" are not limited to the case where the subject (e.g. patient) is cured and the disease is eradicated. Rather, the present invention also contemplates treatment that merely reduces symptoms, and/or delays disease progression.

### DETAILED DESCRIPTION OF THE INVENTION

The present invention provides compositions of fish egg cellular extract and methods for the improved healing of chronic wounds in a diabetic subject. In particular the chronic wounds wounds are associated with diabetes (i.e., diabetic ulcers such as diabetic foot ulcers).

Accordingly, in some embodiments, the present invention provides for the use of an fish egg cellular extract for one or more effects selected from 1) increasing expression and/or release by macrophages of one or more cell mediators selected from the group consisting of IL-1 RA, IL-6, IL-10 and VEGF; 2) decreasing expression and/or release by macrophages of one or more cell mediators selected from the group consisting of IL-12, TNF-α, MCP-1, and IL-8; 3) increasing expression by macrophages of AMAC-1; 4) promoting orientation of macrophage phenotype to an alternatively activated phenotype; and 5) combinations thereof. In further embodiments, the present ivenntion provides for the use of an fish egg cellular extract for promoting, accelerating, or improving healing of chronic wound in a subject. In some embodiments, the chronic wound is a diabetic ulcer.

In some embodiments, 1, 2, 3, or 4 of the following cell mediators are increased, in any combination: IL-1 RA, IL-6, IL-10 and VEGF. In some embodiments, 1, 2, 3, or 4 of the following cell mediators are decreased, in any combination: IL-1 RA, IL-6, IL-10 and VEGF. IL-12, TNF-α, MCP-1, and IL-8. In some embodiments, the ratio of alternatively activated macrophages: classically activated macrophages at the site of the wound is increased upon application of the fish egg cellular extract. In some embodiments, the macrophages are present at the site of a wound. In some embodiments, the alternatively activated phenotype is characterized by expression of Arg1. In some embodiments, the classically activated phenotype is characterized by expression of Nos2.

In some embodiments, the fish egg cellular extract comprises about 100 to 380 mg/ml protein in an aqueous solution; about 0.1 to 10 mg/ml RNA; about 0.1 to 5 mg/ml DNA and 0.1 -10% lipids w/w; wherein the composition has an osmolarity of from about 330 to 440 mOsm, a pH of from about 5.0 to 7.7, and density of from about 0.8 to 1.4 g/ml. In some embodiments, the fish egg cellular extract is selected from the group consisting of an extract of an activated fish egg cellular extract and an unactivated fish egg cellular extract. In some embodiments, the fish egg cellular extract is from a fertilized egg. In some embodiments, the cellular extract is heat treated by heating the extract to greater than 80C, 90C, 95C or 100C. In some embodiments, the heat treatment is from about 1 minute to about 30 minutes. In some embodiments, the fish egg cellular extract is provided in a cream, gel, emulsion, ointment, spray, powder or lotion.

As just described, the compositions of the present disclosure utilize cell, egg and embryo extracts from vertebrates, including but not limited to Superclass Gnathostomata (jawed vertebrates), Euteleostomi (bony vertebrates), Class Actinopterygii (ray-finned fishes), Class Sarcopterygii (lobe-finned fishes and terrestrial vertebrates), Tetrapoda (tetrapods), Amniota (amniotes), Synapsida (synapsids), Class Mammalia (mammals), Early Therapsida (early therapsids), Class Reptilia (reptiles), Anapsida (tortoises and turtles), Order Testudines (tortoises and turtles), Diapsida (birds, crocodiles, lizards, snakes, and relatives), Archosauria (birds and crocodiles), Order Crocodilia (caimans, crocodiles, and relatives), Lepidosauria (amphisbaenians, lizards, snakes, and tuataras), Order Rhynchocephalia (tuataras), Order Squamata (amphisbaenians, lizards, and snakes), Class Amphibia (amphibians), Subclass Dipnoi (lungfishes), Actinistia, Order Coelacanthiformes (coelacanths), Class Chondrichthyes (rays, sharks, and relatives), Placodermi (armored fishes and placoderms), Class Cephalaspidomorphi, more preferably fish, shrimp, sea urchin or amphibian eggs or embryos. In some aspects of the disclosure, unfertilized but activated fish, shrimp, sea urchin or amphibian eggs are used. The present disclosure is not limited to the use of any particular types of eggs. Indeed, the use of a variety of eggs is contemplated, including, but not limited to eggs from Xenopus, shrimp, sea urchin, salmon, trout or zebrafish. In some aspects of the disclosure, eggs are collected from mature females and spontaneously activate upon contact with water. In further aspects, the eggs are washed in Ringer's saline. In some embodiments, the eggs are not from an avian species.

Extracts of the present disclosure are prepared from any of the sources described herein. In some aspects pof the disclosure, the extracts are cellular extracts. Cellular extracts of the present disclosure are preferably compositions of disrupted cells such as eggs. The cells may be disrupted by a variety of methods, including, but not limited to, mechanical shearing or blending, sonication, or osmotic lysis. In some aspects, the cellular extracts are preferably further processed to yield a composition that is substantially free of lipids naturally associated with the cells, such as cell membrane components. By substantially free of lipids, it is meant that the cellular extract comprises less than about 1%, preferably less than about 0.5%, and more preferably less than about 0.1% of lipids that are naturally associated with the cells used to make the cellular extract. In some embodiments, the extracts comprise less than about 1% and preferably less than 0.1% cholesterol or ovalbumin. Accordingly, in some aspects of the disclosure, the cellular extract comprises carbohydrates, proteins, glycosylated or otherwise modified proteins, peptides, amino acids, RNA (mRNA, sRNA, miRNA, rRNA), DNA, water etc, and combinations thereof. In some aspects, the cellular extracts can comprise small amounts of lipids naturally associated with the cells, as well as nuclear components such as chromosomes, nucleic acids, and nuclear proteins. In some aspects, the cellular extract is preferably a cytoplasmic extract or fraction prepared by removing nuclear, cell membrane and other water insoluble materials naturally associated with the cells. In some aspects, these components are removed by centrifugation or fractionation of the disrupted cells. In some aspects, the cellular extract is preferably an aqueous extract or fraction comprising water soluble cellular components such as proteins, mRNA, and carbohydrates.

A variety of methods may be used to prepare extracts. For example, in some embodiments, eggs are placed "dry" in a glass 15 ml centrifuge tube, and crushed by sedimentation at 15,000 g for 15 min. This produces three layers: a lipid top fraction, which is collected, aliquoted and frozen; a middle cellular or cytoplasmic fraction, which is also collected, aliquoted and frozen; and a pellet fraction, which is discarded. In some embodiments, the cellular fraction or extract primarily comprises contents of the cytoplasm. The cellular fraction is used as extract. In some embodiments, the cellular fraction may be used in combination with a lipid fraction. The cytoplasmic fraction may be cleared further by sedimentation at 50,000, 100,000 or 200,000 g to yield a further cellular extract which is primarily a water soluble extract fraction. Regardless of the fraction used, the extract can be diluted to about 300 mOsm with cell lysis buffer (see above), if necessary. Accordingly, in some preferred embodiment's, a water soluble extract prepared from eggs or embryos is utilized.

In other embodiments, the eggs are suspended in 0.5 volume of cell lysis buffer and sonicated on ice until all eggs are lysed. The particulate material is sedimented at 15,000 g for 15 min at 4°C. The supernatant constitutes the extract. As above, osmolarity can be adjusted to 300 mOsm if needed. The extract can also be cleared as above.

In still other embodiments, the eggs are suspended in cell lysis buffer as in method 2. Eggs are lysed by Dounce homogenization using a glass mortar and pestle (Kontes, type A or B). The lysate is sedimented and treated as described above.

In some preferred aspects, the present disclosure provides compositions, either prepared from natural sources as described above or from artificial source materials, or a combination thereof. In some aspects, the extracts are characterized as having an osmolarity of from about 330 to 440, preferably about 350 mOsm. In some aspects, the extracts have a pH of from about 5.0 to about 7.7, preferably a pH of about 6.5 - 7.0. In some aspects, the extracts have a protein content of about 100 to 250 mg/ml, preferably about 160 to 190 mg/ml, and most preferably about 120 mg/ml. In some aspects, the compositions have a water content of about 20 to 90 percent water weight/weight (w/w), preferably about 37 to 79% water w/w. In some aspects, the extracts have a density of about 0.8 to about 1.4 g/ml, preferably about 1.1 g/ml. In some aspects, the compositions comprise trace elements including, but not limited to, calcium, phosphorus, zinc, copper and iron. In some embodiments, the compositions comprise vitamins, including, but not limited to vitamins A, C, E, riboflavin, niacin, B 6 , calcium pantothenate and B 12. In some aspects, the present invention provides a fresh roe composition comprising: 2.7 to 3.4% protein; 3 to 5% carbohydrates; 1.0 to 1.7% fats in the form of phospholipids, and 0.01 to 0.05% minerals in fresh roe, should be less fats and higher total protein in the extract), 37 to 79 weight percent water. In some aspects, the extracts further comprise a lipid fraction. In some aspects, the lipid fraction comprises from about 60% to about 80% unsaturated fatty acids. In further aspects, the compositions comprise phospholipids, including phosphatidyl cholines (lecithins) or as phosphatidyl ethanolamine (cephalins), and to a lesser extent inositol phosphatides, cerebrosides and sphingomyelines. In some embodiments, the lipid fraction is from about 0.1% to about 1%, 2%, 3%, 4% or 5% of the total composition, while in other embodiments, the compositions are substantially free or free of lipids.

In some aspects of the disclosure, the artificial extracts are prepared from 1) water, 2) any type of protein (BSA, albumin, vitellogenin, amino acid mixtures, etc.), 3) vitamins and minerals as described above, 4) salts or osmols to create osmolarity of approx 350 mOsm, 5) glycerol or other agent to increase viscosity, 6) lipids such as lecithins, cephalins and other phospholipids, 7) carbohydrates, 8) growth factors such as FGF, EGF and IGF, 9) and chemo-attractants such asSLC/6Ckine/Exodus2/TCA4 and CKbeta-11/MIP-3beta/ELC, 10) acid or base to adjust pH to 6,2 - 7,2, and 11) preservatives such as methyl paraben, propyl paraben, BHA or BHT.

In some embodiments, the eggs or extracts are treated to prevent bacterial growth. The use of a variety of methods is contemplated. In some embodiments, the following methods are combined. In some embodiments, unfertilized or fertilized eggs (e.g., fish or amphibian eggs) are treated prior to homogenization with a bactericidal or bacteriostatic agent. Preferred agents include, but are not limited to, iodine containing agents such as betadine, buffodine, and povidone-iodine, and other agents such as novasan, sodium hypochlorite, bacitracin, polymyxin B sulfate, silver containing compounds such as silver sulfadiazine and silver nitrate, mafenide acetate, nystatin, gentamicin, neomycin. In other embodiments, the extracts are treated post-homogenization to prevent bacterial growth. In some embodiments, the extracts, such as the cellular extracts or cytoplasmic fractions, are treated by heating. In some embodiments, the extracts are heated to about 37, 40, 50, 60, 70, 80 or 90 degrees Celsius for about 30 seconds or 1, 2, 5, 10, 20, 30, 60 or 120 minutes.

In some embodiments, the eggs or extracts are filtered, preferably through 0.22 or 0.45um filters to remove bacteria. In some embodiments, before or after filtering, the extracts are treated by additional centrifugation (15 min - 2 hrs) after heating the extract (to about to about 37, 40, 50, 56 60, 70, 80, 90 or 95 degrees Celsius for about 30 seconds or 1, 2, 5, 10, 20, 30, 60 or 120 minutes) to spin down any bacteria present. It has surprisingly been found that the biological activity of the extracts is retained even after aggressive heat treatment. In some embodiments, the retained biological activity is the ability to stimulate mitochondrial activity and/or the ability to increase hyaluronic acid production in skin fibroblasts.

In other embodiments, eggs are washed in a sulfur-containing agent (e.g., calcium polysulfide or calcium thiosulfate (lime sulfur)) prior to preparation. In some embodiments, sulfur is added to the extracts to remove bacteria. In other embodiments, benzoyl peroxide is added to the extracts. In some embodiments, eggs are washed in 0.001% to about 0.2% by weight of a metal chlorite and sufficient acid to adjust the pH of the solution from about 2.2 to about 4.5 to remove bacteria. In further embodiments, the eggs and/or extract are placed in a vacuum drum and mixed with a natural solution containing salt, vitamin C or citric acid, and water to remove bacteria. In some embodiments, the eggs and/or extract are stirred, vortexed, sonicated, agitated or shaken with salt water or liquid buffer to dislodge bacteria and vacuum filter off the liquid to remove bacteria. It will be possible to check bacterial content in the liquid and on the treated eggs for quality control. In some embodiments, electrophoresis of the eggs and/or extract is used to remove bacteria. It is contemplated that such methods utilize the influences of electrical double layer, intensity of electrical field, electric density gradient, pH of the buffer solution, ionic strength of buffer solution, stage of growth of bacteria, and anion surface-active agent upon the electrophoretic mobility of some species of bacteria.

In some embodiments, lipids are removed by treatments the homogenate prior to centrifugation or the extract after centrifugation. The use of a variety of methods is contemplated. In some embodiments, lipids are removed by filtering through fat-absorbing paper or filter by applying a vacuum suction system to a container with a filter in the bottom, where the extract is placed in the container and suctioned through the filter. In some embodiments, lipids are removed by using an absorbent material and an outer containment vessel. The extract is entered to a container filled with absorbent material through a pump and then recovered by applying a vacuum. In some embodiments, lipids are removed with hollow fiber contraction systems and/or extraction solvents for removing lipids from viscous fluids, where contact a fluid with an extraction solvent, which causes the lipids in the fluid to separate from the fluid or causes lipids in the lipid-containing organisms to separate from the lipid-containing organism, using at least one hollow fiber contactor.

In some embodiments, the homogenates and extracts may be stabilized by the addition of one or more stabilizing agents, such as a lipid stabilizing agent, or by packaging in a package designed to prevent oxidation. In some embodiments, antioxidants such as vitamin E are added to the extract to reduce rate of lipid oxidation. In some embodiments, the extracts are packaged in a container under an inert atmosphere. In some embodiments, the extract is packaged to reduce rate of lipid oxidation in air-free containers such as aluminum coated bags (less than 10 kg per bag for efficient removal of oxygen), or containers filled with nitrogen to remove oxygen. In other embodiments, the extracts are packaged in vacuum packed containers with a pump delivery system.

In some embodiments, the fish eggs are treated to prevent bacterial growth as described above. The fish eggs are then homogenized by subjecting the fish eggs to a pressure treatment. In some embodiments, the eggs are subjected to a pressure of from about 1 ton to about 100 tons, preferably about 5 tons to about 50 tons, more preferably about 10 tons to about 30 tons and most preferably about 20 tons. In some embodiments, the pressure is applied via a hydropress. Suitable hydropresses are available from Speidel. In some embodiments, components of the homogenate are separated. In some preferred embodiments, an aqueous cytoplasmic fraction is obtained that comprises protein, DNA, RNA, and other components as described in more detail elsewhere herein. In some embodiments, the extracts comprise a lipid component in addition to the water soluble components. In some embodiments, the extract is separated from the homogenate by centrifugation. In some embodiments, the centrifugation is a continuous-feed process facilitated by a separator. Suitable separators are available, for instance, from GEA Westfalia.

In some embodiments, the present invention provides processes for producing an active fish egg fraction comprising milling the eggs between two or more surfaces disposed so as to cause crushing of the eggs as the eggs pass the surfaces. In some embodiments, at least one of the surfaces comprises cutting elements, for example knurls. In some embodiments, the mill is a roller mill comprising two or more cylindrical rollers. In some embodiments, each of the rollers comprising a cutting surface, for example a knurled surface. In some embodiments, the eggs are placed in a hopper and fed to the milling surface(s). In the case of a roller mill, the eggs are passed between the rollers, causing the eggs to be crushed to form a homogenate. In some embodiments, the milling surfaces (e.g., the surfaces of rollers) are spaced apart to effectuate crushing of eggs passing the surfaces. For example, in some embodiments, the surfaces are separated by from 0.1 to 5 mm, preferably about 0.5 to 2 mm. In some preferred embodiments, the processes further comprise separating an active fraction from said fish egg homogenate, wherein the active fraction comprises about 100 to 380 mg/ml protein in an aqueous solution; about 0.1 to 10 mg/ml RNA; about 0.1 to 5 mg/ml DNA and 0.1 -10% lipids w/w. In some embodiments, the separating of the active fraction is by a centrifugal force, as described above.

In some embodiments, the fish cellular extracts described above, and most preferably the middle fractions, are further fractionated. A variety of method may be used, including, but not limited to, FICOL gradients, gradient centrifugation, protein precipitation, freeze drying, column chromatography, such as size exclusion chromatography and affinity chromatography, gel separation, high pressure liquid chromatography, ChIP, and immunoprecipitation. It will be recognized that these fraction steps yield corresponding fractions such as freeze dried fractions, affinity chromatography fractions, precipitated fractions, etc.

Accordingly, in some embodiments, the present invention provides powders prepared from the fish cellular extracts described above. In some embodiments, the cellular extracts used in the production of the powders are prepared from salmonid eggs. In some embodiments, the cellular extracts used in the production of the powders are prepared from salmon or trout eggs. In some embodiments, the powders are biologically active. In some preferred embodiments, the powders are freeze-dried. In some embodiments, the powders have less than about 10% moisture and most preferably less than about 5% moisture; protein in a concentration of from about 500 to about 800 mg/g powder, preferably from about 600 to about 700 mg/g powder, most preferably about 640 mg/g powder; DNA in a concentration of from about 1 to about 50 µl/mg powder, preferably from about 5 to about 25 µl/mg powder, and most preferably about 16 µl/mg powder; total RNA (e.g., including mRNA, rRNA, and microRNA) in a concentration of from about 1 to about 50 µl/mg powder, preferably from about 5 to about 20 µl/mg powder, and most preferably about 12 µl/mg powder; and lipids in a concentration of from about 100 to about 200 mg/g powder, most preferably about 150 mg/g powder. The powders may preferably be used to make the formulations described herein as an alternative to the non-powdered cellular extracts.

In some embodiments, the fractions are then combined with or resolubilized with components suitable for preparing compositions for topical administration as described in more detail below.

In some embodiments, the fish egg cellular extracts described above (or components of the extracts) are formulated for delivery by a variety of methods. In some embodiments, the extracts described above are formulated for delivery to skin, gastrointestinal tractus, fat deposits, cartilage, bone, connective tissue, muscle or internal organs. In some embodiments, the extracts or components thereof are formulated for oral administration with or without suitable carriers such as starch, sucrose or lactose in tablets, pills, dragees, capsules, solutions, liquids, slurries, suspensions and emulsions. In some embodiments, the oral delivery vehicle comprises an enteric coating. In other embodiments, the extracts or components thereof are formulated for rectal administration as a capsule, cream, suppository or liquid. In some embodiments, the extracts of components thereof are injected by syringe to the peritoneal cavity or into internal organs or tissues. In some embodiments, the extracts or components thereof are formulated for delivery an osmotic pump.

In still other embodiments, the fish egg cellular extracts or components thereof are delivered by microinjection, preferably via particle bombardment (*i.e.,* with a gene gun). Particle mediated gene transfer methods are known in the art, are commercially available, and include, but are not limited to, the gas driven gene delivery instrument descried in McCabe, U.S. Pat. No. 5,584,807. This method involves coating the nucleic acid sequence of interest onto heavy metal particles, and accelerating the coated particles under the pressure of compressed gas for delivery to the target tissue. Other particle bombardment methods are also available. Generally, these methods involve depositing the extract or components thereof upon the surface of small, dense particles of a material such as gold, platinum, or tungsten. The coated particles are themselves then coated onto either a rigid surface, such as a metal plate, or onto a carrier sheet made of a fragile material such as mylar. The coated sheet is then accelerated toward the target biological tissue. The use of the flat sheet generates a uniform spread of accelerated particles that maximizes the number of cells receiving particles under uniform conditions, resulting in the introduction of the nucleic acid sample into the target tissue. This invention contemplates the described use of gene-gun to deliver extracts or components of extracts as defined above.

In still other embodiments, the fish egg cellular extracts or components are microencapsulated (e.g., with collagen or glycosaminoglycans), formed into nanoparticles (e.g., lecithin encapsulated in an oil core), liposomes, microemulsions, or nanoemulsions, oil bodies, retinol molecular fluid films, unilamellar vesicles, multilamellar vesicles, preloaded spherical beads or sponges, elastic vesicles, etc.

In some embodiments, the fish egg cellular extracts, powders or components thereof described above are combined with additional components. In some embodiments, these additional components enhance uptake, bioavailability or penetration of the extract components. In preferred embodiments, extract components may contain natural or a mixture of synthetic components. The components may be partially or totally synthetic. In some embodiments, the cell or extract or synthetic components made from substances identified in the extracts are mixed with a composition comprising water, sebaceous and epidermal lipids and cell extracts, proteins, and components thereof, preferably comprises about a 10% lipid fraction by weight, about a 10% protein fraction by weight, and about an 80% volatile fraction by weight.

In some embodiments, the compositions of the present invention further comprise lipids, preferably lipids having beneficial effects in skin. The lipids include but are not limited to omega-3 fatty acids, myristic acid, and stearidonic acid, and combinations thereof. In some embodiments, the omega-3 fatty acid is eicosapentaenoic acid (EPA), which has been shown to have a photoprotective and anti-aging effect on skin (Kim HH. et al. Photoprotective and anti-skin-aging effects of eicosapentaenoic acid in human skin in vivo. J Lipid Res. 2006 May;47(5):921-30.) In other embodiments, the omega-3 fatty acid is docosapentaenoic acid (DPA), which is beneficial for the cell membrane in fibroblast skin cells. In other embodiments, the omega-3 fatty acid is docosahexaenoic acid DHA). In some embodiments, the lipids comprise a combination of one or more these omega-3 fatty acids. Stearidonic acid reduces skin redness. Myristic acid increases penetration of active substances into skin, specifically into fibroblasts (Eric R. Brown and Papasani V. Subbaiah. Differential effects of eicosapentaenoic acid and docosahexaenoic acid on human skin fibroblasts. Lipids. 1994 December 29(12): 803-913); Zulfakar M.H. et al. Enhanced topical delivery and ex vivo anti-inflammatory activity from a betamethasone dipropionate formulation containing fish oil. J Inflammation Res. Volume 59 (1): 1-88); Mittal A, et al. The effect of penetration enhancers on permeation kinetics of nitrendipine in two different skin models. Biol Pharm Bull 2008 Sep;31(9):1766-72.).

In further embodiments, the compositions further comprise natural marine-derived fat-soluble vitamins such as Vitamin A and E. These are well known to be beneficial for skin both as antioxidants and by direct effect on fibroblast health. Vitamin E is vital in protecting skin cells from ultra violet light, pollution, drugs, and other elements that produce cell damaging free radicals (Riedel SB et al.Vitamin E analog, alpha-tocopherol ether-linked acetic acid analog, alone and in combination with celecoxib, reduces multiplicity of ultraviolet-induced skin cancers in mice. Anticancer Drugs.2008 Feb;19(2):175-81). Vitamin A has been shown in many studies to prevent and reverse cancerous changes in cells in some parts of the body, including the skin, and repairs skin damage caused by the sun (Alberts D. et al. Safety and efficacy of dose-intensive oral vitamin A in subjects with sun-damaged skin. Clinical Cancer Research 2004;10:1875-80.).

In further embodiments the compositions comprise natural or synthetic proteins including but not limited to the protein vitellogenin, known for its rejuvenating properties. Vitellogenin is also present in honey, heralded for giving bees prolonged lives (Miinch D, Amdam GV. The curious case of aging plasticity in honey bees. FEBS Lett. 2010 Apr 10).Ref 9) In further embodiments the composition contains natural or synthetic peptides, herein defined as polymers formed from the linking, in a defined order, of α-amino acids; including but not limited to milk peptides, ribosomal peptides, nonribosomal peptides, peptones and peptide fragments. Peptides are believed to have a good effect on skin and wrinkles. Because peptides are so small, it is thought that they may more easily penetrate the skin and yield their effects.

In some embodiment, the invention relates to compositions where the lipid fraction preferably comprises components in vernix, i.e., lecithin and other phospholipids, squalene, waxes, wax esters, sterol esters, diol esters, triglycerides, free sterols and four classes of fatty acids ranging in chain length from C₁₂ to C₂₆ (straight chain saturated, straight chain unsaturated, branched chain saturated, and branched chain unsaturated). In preferred embodiments, the vernix lipid components are as follow, with the relative percentages indicated, squalene (9%), aliphatic waxes (12%), sterol esters (33%), diesters (7%), triglycerides (26%), free sterols (9%), other lipids (4%). In additional embodiments, the lipid composition is composed of lipids from egg and/or fish roe with wound healing properties 30% of which are barrier lipids (proteolipid matrix); cholesterol (1.1 %, 52.8 % of barrier), free fatty acids (0.6%, 27.7% of barrier), phospholipids (0.4%), ceramides (0.7 %, 20.1% barrier). In another preferred embodiment, the protein fraction contains the protein components of vernix, i.e., keratin, filaggrin, regulator proteins (e.g. EGF), and glutamine.

The fatty acids within the aliphatic waxes may be branched and the branched fatty acids may be methylated. The protein fraction consists of epidermally derived proteins, primarily keratin and filaggrin. The protein fraction also contains trace amounts in the range of about micromolar to millimolar concentrations of regulatory proteins such as epidermal growth factor (EGF), and trace amounts of about nanomolar to micromolar concentrations of surfactant protein such as Surfactant A and Surfactant B. The volatile fraction is primarily water. The rate of evaporation of volatile components is relatively slow, presumably due to increased energy requirements for the dissociation of hydrogen bonds and for diffusion from the cellular component through the lipid component to change water from the liquid to the gaseous state.

In some embodiments, the fish egg cellular extracts or components are combined with phospholipids or other lipophilic substances, palmitylmyristrates, dimethylsulfoxide (DMSO), chitosan, long chain organic polymers such as polysaccharides, non-aqueous solvents, beta-glucan, pH adjusting components, skin metabolism inhibition agents, propylene glycol, butylenes glycol, polyethylene glycol, olive oil or other naturally occurring oils, dimethyl isosorbide, dimethylformamide, methyl salicylate, long chain oleic acid, mucopolysaccharides, and other agents.

In some embodiments, the additional agents include, but are not limited to, ubiquitin, antimicrobial agents (alpha-defensins, LL37, beta-defensins, etc.), surfactant proteins from the collectin family (collecting associated protein A and D), nicotinamide and psoriacin.

In some embodiments, the additional agents include, but are not limited to, vitamins, antioxidants, minerals, extracts, and chemical compounds such as alpha-tocopherol (vitamin E), melanin, vitamin C, provitamin A, retinyl proprionate, retinoic acid, Vitamin D3, Nicotinamide (vitamin B), Niacinaminde (Vit B3, exfoliates surface skin), d-panthenol (aids in skin repair of damage), vitamin A, hyaluronic acid, ceramides, Seaweed (algae) Mineral oil (paraffinum liquidium) Petrolatum Glycerin Isohexadecane Cirtus aurantifolia (lime) extract Microcrystalline wax (cera microcristallina) Lanolin alcohol Seamum indicium (sesame) seed oil, Eucalyptus globules (eucalyptus) leaf oil, Magnesium sulfate, Sesamum indicum (sesame) seeds, Medicago satvia (alfalfa) seeds, Helianthus annuus (sunflower) seeds, Prunus dulcis (powdered almonds), Sodium, Potassium, Copper, Calcium, Magnesium, zinc gluconate, Paraffin, Vitamin E succinate, Niacin, Beta-carotene, Decyl oleate, Aluminum distearate, Octyuldodecanol, Citric acid, Cyanocobalamin, Magnesium stearate, Panthenol, Limonene, Geraniol, Linalool, Hydroxycitronellal, Citronellol, Benzyl salicylate, Citral, Methylchloroisothiazoline, Methylisothiazolinone, Alcohol denat., Fragrance (parfum), Butylene glycol, Byrospermum parkii (shea butter), Fish (pisces) cartilage extract, Polyethylene, Hydrogenated polyisobutene, Cyclopentasiloxane, Cetyl esters, Cetearyl alcohol, Malachite, Isostearyl neopentanoate, Polybutene, Sucrose, Silica, Tocotrienol, Cucumis satvius (cucumber) fruit extract, Centella asiatica (hydrocotyl) extract, Seamum indicium (sesame) seeds, Eucalyptus globules (eucalyptus) leaf oil, Medicago satvia (alfalfa) seeds, Helianthus annuus (sunflower) seeds, Prunus dulcis (powdered almonds), Potassium, Copper, Calcium, Magnesium, Caffeine, Sodiumhyaluronate, Linoleic acid, Cholesteryl/behenyl/octyldodecyl lauroyl glutamate, Methyl glucose sesquisterate, Cholesterol, Dimethicone, Ocimum basilicum (basil), Mentha arvensis (wild mint), Acrylates/C10-30 alkyl acrylate crosspolymer, Glyceryl distearate, Cetearyl glucoside, Steareth-10, Carbomer, Aminomethyl propanol, Limonene, Linalool, Benzyl salicylate, Disodium EDTA, BHT, Sodium dehydroacetate, Phenoxyethanol, Methylparaben, Titanium dioxide (CI 77891), C12-20 acid PEG-8 Ester, Hydrogenated vegetable oil, Petrolatum, Butylene Glycol, Glycerin, Acetylated Lanolin, Glycoproteins, Panax, Ginseng Root extract, Equisetum Arvense (Horsetail) Extract, Sodium carbomer, Beeswax (cera alba), Cetyl phosphate, Polyperfluoromethylisoporpyl ether, Benzyl alcohol, Linalool, Hydroxycitronellal, Alpha-isomethyl ionone, Amyl cinnamal, Hexyl cinnamal, Verenia furfuracea (treemoss) extract, Geraniol, Benzyl benzoate, Bytulphenol methylpropional, Eugenol, Benzyl salicylate, Chlorphenesin, Phenoxyethanol, and Methylparaben.

In some embodiments, the compositions of the present invention are useful for facilitating the delivery of active compounds via the skin. In some preferred embodiments, one or more active agents, such as a protein, small organic compound, or one of the agents identified above are combined with the cytoplasmic fraction of, for example, a fertilized or unfertilized amphibian or fish eggs. Cytoplasmic fractions and method for making such fractions are disclosed elsewhere in the application in detail. Accordingly, in some embodiments, the present invention provides compositions comprising a cytoplasmic fraction of amphibian or fish eggs and one or more active agents. In some embodiments, the present invention provides methods of facilitating the penetration of one or more active agents into the skin, comprising providing a composition comprising a cytoplasmic extract from amphibian and/or fish eggs and one or more active agents and contacting the skin of a subject with the composition. As described above, the composition can be preferably be an emulsion, salve, cream, gel, spray, aerosol, liquid, etc.

Exemplary proteins that can be active agents include, but are not limited to, Alzheimer's amyloid peptide (Aβ), SOD1, presenillin 1 and 2, renin, α-synuclein, amyloid A, amyloid P, activin, anti-HER-2, bombesin, enkephalinase, protease inhibitors, therapeutic enzymes, α1-antitrypsin, mammalian trypsin inhibitor, mammalian pancreatic trypsin inhibitor, calcitonin, cardiac hypertrophy factor, cardiotrophins (such as cardiotrophin-1), CD proteins (such as CD-3, CD-4, CD-8 and CD-19), CFTR, CTNF, DNase, human chorionic gonadotropin, mouse gonadotropin-associated peptide, cytokines, transthyretin, amylin, lipoproteins, lymphokines, lysozyme, a growth hormone (including human growth hormone), bovine growth hormone, growth hormone releasing factor, parathyroid hormone, thyroid stimulating hormone, growth factors, brain-derived neurotrophic growth factor, epidermal growth factor (EGF), fibroblast growth factor (such as α FGF and β FGF), insulin-like growth factor-I and -II, des(1-3)-IGF-I (brain IGF-I), insulin-like growth factor binding proteins, nerve growth factor (such as NGF-β), platelet-derived growth factor (PDGF), vascular endothelial growth factor (VEGF), receptors for growth hormones or growth factors, transforming growth factor (TGF) (such as TGF-α, TGF-β1, TGF-β2, TGF-β3, TGF-β4 or TGF-β5), neurotrophic factors (such as neurotrophin-3, -4 ,-5, or -6), gelsolin, glucagon, kallikreins, mullerian-inhibiting substance, neurotrophic factors, p53, protein A or D, prorelaxin, relaxin A-chain, relaxin B-chain, rheumatoid factors, rhodopsin, a serum albumin (such as human serum albumin), inhibin, insulin, insulin chains, insulin A-chain, insulin β-chain, insulin receptor, proinsulin, luteinizing hormone, integrin, interleukins (ILs) (such as IL-1 to IL-10, IL12, IL-13), erythropoietin, thrombopoietin, fibrillin, follicle stimulating hormone, clotting factors (such as factor VIIIC, factor IX, tissue factor, and von Willebrands factor, anti-clotting factors (such as Protein C, atrial naturietic factor, lung surfactant), a plasminogen activator (such as human tissue plasminogen activator or urokinase), thrombin, tumor necrosis factor-α or β, α-ketoacid dehydrogenase, addressins, bone morphogenetic proteins (BMPs), collagen, colony stimulating factors (CSFs) (such as M-CSF, GM-CSF and G-CSF), decay accelerating factor, homing receptors, interferons (such as interferon-α, -β and -y), keratin, osteoinductive factors, PRNP, regulatory proteins, superoxide dismutase, surface membrane proteins, transport proteins, T-cell receptors, viral antigens such as a portion of the AIDS envelope, immunoglobulin light chain, antibodies, antibody fragments (such as single-chain Fv fragment (scFv), single-chain antibody (scAb), F_{AB} antibody fragment, diabody, triabody, fluorobody), antigens such as gp120(IIIb) immunotoxins, atrial natriuretic peptide, seminal vesicle exocrine protein, β2-microglobulin, PrP, precalcitonin, ataxin 1, ataxin 2, ataxin 3, ataxin 6, ataxin 7, huntingtin, androgen receptor, CREB-binding protein, gp120, p300, CREB, AP1, ras, NFAT, jun, fos, dentaorubral pallidoluysian atrophy-associated protein, a microbial protein (e.g., maltose binding protein, ABC transporter, glutathione S transferase, thioredoxin, β-lactamase), green fluorescent protein, red fluorescent protein, or derivatives or active fragments or genetic variants of any of the peptides listed above.

Examples of small organic compounds include, but are not limited to, non-steroidal anti-inflammatory drugs (NSAIDS)(the NAIDS can, for example, be selected from the following categories: *(e.g.,* propionic acid derivatives, acetic acid derivatives, fenamic acid derivatives, biphenylcarboxylic acid derivatives and oxicams)); steroidal anti-inflammatory drugs including hydrocortisone and the like; antihistaminic drugs *(e.g.,* chlorpheniranune, triprolidine); antitussive drugs *(e.g.,* dextromethorphan, codeine, carmiphen and carbetapentane); antipruritic drugs *(e.g.,* methidilizine and trimeprizine); anticholinergic drugs *(e.g.,* scopolamine, atropine, homatropine, levodopa); anti-emetic and antinauseant drugs *(e.g.,* cyclizine, meclizine, chlorpromazine, buclizine); anorexic drugs *(e.g.,* benzphetamine, phentermine, chlorphentermine, fenfluramine); central stimulant drugs *(e.g.,* amphetamine, methamphetamine, dextroamphetamine and methylphenidate); minoxidil; antiarrhythmic drugs *(e.g.,* propanolol, procainamide, disopyraminde, quinidine, encainide); P-adrenergic blocker drugs *(e.g.,* metoprolol, acebutolol, betaxolol,labetalol and timolol); cardiotonic drugs *(e.g.,* milrinone, amrinone and dobutamine); antihypertensive drugs *(e.g.,* enalapril, clonidine, hydralazine, minoxidil, guanadrel, guanethidine);diuretic drugs *(e.g.,* amiloride and hydrochlorothiazide); vasodilator drugs *(e.g.,* diltazem, amiodarone, isosuprine, nylidrin, tolazoline and verapamil); vasoconstrictor drugs *(e.g.,* dihydroergotamine, ergotamine and methylsergide); antiulcer drugs *(e.g.,* ranitidine and cimetidine); anesthetic drugs *(e.g.,* lidocaine, bupivacaine, chlorprocaine, dibucaine); antidepressant drugs *(e.g.,* imipramine, desipramine, amitryptiline, nortryptiline); PDE5 inhibitors such as Viagra^{®} or Cialis^{®}; tranquilizer and sedative drugs *(e.g.,* chlordiazepoxide, benacytyzine, benzquinamide, flurazapam, hydroxyzine, loxapine and promazine); antipsychotic drugs *(e.g.,* chlorprothixene, fluphenazine, haloperidol, molindone, thioridazine and trifluoperazine); antimicrobial drugs (antibacterial, antifungal, antiprotozoal and antiviral drugs).

Antimicrobial drugs which are preferred for incorporation into the present composition include, for example, pharmaceutically acceptable salts of β-lactam drugs, quinolone drugs, ciprofloxacin, norfloxacin, tetracycline, erythromycin, amikacin, triclosan, doxycycline, capreomycin, chlorhexidine, chlortetracycline, oxytetracycline, clindamycin, ethambutol, hexamidine isothionate, metronidazole; pentamidine, gentamycin, kanamycin, lineomycin, methacycline, methenamine, minocycline, neomycin, netilmycin, paromomycin, streptomycin, tobramycin, miconazole, and amanfadine.

Other drug moieties of use in practicing the present invention include antineoplastic drugs (*e.g.,* antiandrogens *(e.g.,* leuprolide or flutamide), cytocidal agents *(e.g.,* adriamycin, doxorubicin, taxol, cyclophosphamide, busulfan, cisplatin, a-2-interferon) anti-estrogens *(e.g.,* tamoxifen), antimetabolites *(e.g.,* fluorouracil, methotrexate, mercaptopurine, thioguanine).

The compositions can also comprise hormones *(e.g.,* medroxyprogesterone, estradiol, leuprolide, megestrol, octreotide or somatostatin); muscle relaxant drugs *(e.g.,* cinnamedrine, cyclobenzaprine, flavoxate, orphenadrine, papaverine, mebeverine, idaverine, ritodrine, dephenoxylate, dantrolene and azumolen); antispasmodic drugs; bone-active drugs *(e.g.,* diphosphonate and phosphonoalkylphosphinate drug compounds); endocrine modulating drugs *(e.g.,* contraceptives *(e.g.,* ethinodiol, ethinyl estradiol, norethindrone, mestranol, desogestrel, medroxyprogesterone), modulators of diabetes *(e.g.,* glyburide or chlorpropamide), anabolics, such as testolactone or stanozolol, androgens *(e.g.,* methyltestosterone, testosterone or fluoxymesterone), antidiuretics *(e.g.,* desmopressin) and calcitonins).

Also of use in the present invention are estrogens *(e.g.,* diethylstilbesterol), glucocorticoids *(e.g.,* triamcinolone, betamethasone, *etc*.) and progenstogens, such as norethindrone, ethynodiol, norethindrone, levonorgestrel; thyroid agents *(e.g.,* liothyronine or levothyroxine) or anti-thyroid agents *(e.g.,* methimazole); antihyperprolactinemic drugs *(e.g.,* cabergoline); hormone suppressors *(e.g.,* danazol or goserelin), oxytocics *(e.g.,* methylergonovine or oxytocin) and prostaglandins, such as mioprostol, alprostadil or dinoprostone, can also be employed.

Other useful active compounds include immunomodulating drugs (*e.g.,* antihistamines, mast cell stabilizers, such as lodoxamide and/or cromolyn, steroids (e.g., triamcinolone, beclomethazone, cortisone, dexamethasone, prednisolone, methylprednisolone, beclomethasone, or clobetasol), histamine H₂ antagonists *(e.g.,* famotidine, cimetidine, ranitidine), immunosuppressants *(e.g.,* azathioprine, cyclosporin), etc. Groups with anti-inflammatory activity, such as sulindac, etodolac, ketoprofen and ketorolac, are also of use. Other drugs of use in conjunction with the present invention will be apparent to those of skill in the art.

In some embodiments, components of the fish egg cellular extract may act as chemotaxants. Mesenchymal stem cells and fibrocytes circulates in the blood stream and in case of skin wound they penetrate the wound area where they can differentiate to skin cells like fibroblasts, keratinocytes, pericytes, adipose and endothelial cells. Chemotaxants in the extract may act as ligands for the CCR7 involved in attractin immune cells and dendritic cells and may include SLC/6Ckine/Exodus2/TCA4 and CKbeta-11/MIP-3beta/ELC

In some embodiments, the fish egg cellular extracts and powders described above (or components of the extracts and powders) are formulated for topical delivery. General formulations for topical delivery are described in Remington's Pharmaceutical Sciences, 18th Edition, Mack Publishing, p. 1288-1300 [1990]. Accordingly, in some embodiments, the extracts are formulated as a water based gel or paste, ointment, cream (anhydrous or hydrous), lotion (anhydrous or hydrous), emulsion, spray, solution, aerosol, stick (solid cream), liquid band aid, powder, inhalation spray, nasal spray, basal drops, cheek drops, sublingual drops, eye drops or sprays, ear drops or sprays, and transdermal patches.

It is contemplated that the compositions for topical application find use for both cosmetic and therapeutic purposes. In some embodiments, it is contemplated that the compositions described above are applied directly to the skin or other epithelial or epidermal surfaces of the body. The compositions may be applied one, two, three or more times each day as is appropriate for the indication. The amount applied is not generally important, but generally a composition comprising from about 0.001 µg to 10 grams of the extract or powder (or components thereof) may be applied to a given surface of the body. As described above, the composition may comprise other components such as adjuvants, carriers, other active ingredients, etc.

In some embodiments, the invention relates to compositions that include preservatives and antioxidants (including vitamins) to prevent product deterioration preferably trisodium and tetrasodium edetate (EDTA) and tocopherol (vitamin E). In further embodiments the composition contains antimicrobials to fight bacteria preferably butyl, propyl, ethyl, and methyl parabens, DMDM hydantoin, methylisothiazolinone phenoxyethanol (also rose ether fragrance component), quaternium-15. In further embodiments, the composition contains thickeners and waxes used in stick products such as lipsticks and blushers preferably candelilla, carnauba, and microcrystalline waxes carbomer and polyethylene-thickeners. In further embodiments, the composition contains solvents to dilute preferably butylene glycol and propylene glycol, cyclomethicone (volatile silicone), ethanol (alcohol) and glycerin. In further embodiments, the composition contains emulsifiers to break up and refine preferably glyceryl monostearate (also pearlescent agent), lauramide DEA (also foam booster) and polysorbates. In some embodiments, the compositions contain color additives--synthetic organic colors derived from coal and petroleum sources preferably D&C Red No. 7 Calcium Lake (and other dyes that do not dissolve in water), iron oxides, mica (iridescent), and aminophenols. In further embodiments, the compositions contain pH adjusters to stabilize or adjust acids and bases preferably ammonium hydroxide--in skin peels and hair waving and straightening, citric acid--adjusts pH, and triethanolamine--pH adjuster used mostly in transparent soap. In further embodiments, the compositions contains agents preferably magnesium aluminum silicate--absorbent, anti-caking agent, silica (silicon dioxide)-absorbent, anti-caking, abrasive, sodium lauryl sulfate-detergent, stearic acid--cleansing, emulsifier, talc (powdered magnesium silicate)--absorbent, anti-caking, and zinc stearateused in powder to improve texture, lubricates.

The composition includes the recited components (e.g., cellular extracts or powders) and combinations thereof in a total amount of about 0.5 to 50 grams per liter or about 0.5 to 50 grams per 1000 grams of the formuation, and more preferably about 1 to 10 grams per liter or about 1 to 10 grams per 1000 grams of the formulation, although higher or lower concentrations are permissible. Such compositions being in the form of an emulsion, cream, salve or the like, the active materials being admixed with water, alkylene glycols, various oils natural and synthetic, petrolatum, preservatives, coloring agents, perfumes, and like ingredients conventional in the cosmetic arts.

The composition can be applied to the face, eyelids, skin mouth, mucosal membranes or other body parts in an amount varying with the individual. About 0.01 to 1, advantageously about 0.02 to 0.75 and preferably about 0.3 to 0.5, grams per cm² has been found useful but more or less can be used. The application can be once weekly or more often, even several times a day.

In accordance with the compositions and method of the present invention, the egg cell extracts of the present invention may be administered in the form of a pharmaceutical composition additionally comprising a pharmaceutically acceptable carrier. One skilled in the art will appreciate that suitable methods of administering the extract compositions to an animal, such as a mammal, are available and, although more than one method can be used to administer a particular composition, a particular method and dosage can provide a more immediate and more effective reaction than others. Pharmaceutically acceptable carriers are also well known to those skilled in the art. The choice of carrier will be determined, in part, both by the particular composition and by the particular method used to administer the composition. Accordingly, there is a wide variety of suitable formulations of the pharmaceutical compositions of the present invention.

In some preferred embodiments, the formulations of this invention are designed for topical administration. Typical of such formulations are ointments, creams, and gels.

Ointments generally are prepared using either (1) an oleaginous base, i.e., one consisting of fixed oils or hydrocarbons, such as white petrolatum or mineral oil, or (2) an absorbant base, i.e., one consisting of an anhydrous substance or substances which can absorb water, for example, anhydrous lanolin. Customarily, following formation of the base, whether oleaginous or absorbent, the active ingredient (e.g., salmon egg extract) is added in an amount affording the desired concentration.

Creams are oil/water emulsions. They consist of an oil phase (internal phase), comprising typically fixed oils, hydrocarbons, and the like, such as waxes, petrolatum, mineral oil, and the like, and an aqueous phase (continuous phase), comprising water and any water-soluble substances, such as added salts. The two phases are stabilized by use of an emulsifying agent, for example, a surface active agent, such as sodium lauryl sulfate; hydrophilic colloids, such as acacia colloidal clays, veegum, and the like. Upon formation of the emulsion, the active ingredient (e.g., salmon egg extract) customarily is added in an amount to achieve the desired concentration.

Gels comprise a base selected from an oleaginous base, water, or an emulsion-suspension base, such as described above. To the base is added a gelling agent which forms a matrix in the base, increasing its viscosity. Examples of gelling agents are hydroxypropyl cellulose, acrylic acid polymers, and the like. Customarily, the active ingredient (IGF-II) is added to the formulation at the desired concentration at a point preceding addition of the gelling agent.

Serums may be watery or thicker liquids, often (but not always) clear in color. Serums are water based making them light in consistency. They are easily and quickly absorbed into the skin and provide an excellent way to deliver topical ingredients including Vitamin C, peptides, alpha hydroxy acids, retinols. Serums may be layered under other serums as well as creams or lotions making them a very flexible product to incorporate into your skin care regimen. Serums are tolerated well by all skin types as long as the individual is not sensitive to any of the ingredients. Serums may include glycerol or glycerine. The amount of extract incorporated into the formulation of this invention is not critical; the concentration should only be in a range sufficient to permit ready application of the formulation to the wound area in an amount which will deliver the desired amount of extract.

The customary amount of formulation to be applied will depend upon concentration of the active ingredient in the formulation. In some embodiments, the amount of protein in the extract is determined. Then, a specific amount of the extract or powder is included in the pharmaceutically acceptable carrier based on the amount of protein. Generally, the formulation will be applied to the wound in an amount affording from about 0.1 to about 500 µg of protein per cm² of skin. Preferably, the applied amount of protein will range from about 1 to about 300 µg/cm², more preferably, from about 5 to about 200 µg/cm². In other embodiments, a specific volume of extract is added to the pharmaceutically acceptable carrier or other excipient. Accordingly, in some embodiments, the compositions of the present invention comprise on a volume/volume basis (volume of extract per total volume of the formulation), for example, from about 0.001 to 50% extract, about 0.01 to 50% extract, about 0.1 to 50% extract, about 0.001 to 10% extract, about 0.01 to 10% extract, about 0.1 to 10% extract, about 0.001 to 5% extract, about 0.01 to 5% extract, about 0.1 to 5% extract, about 0.001 to 4% extract, about 0.01 to 4% extract, about 0.1 to 4% extract, about 0.001 to 2% extract, about 0.01 to 2% extract, about 0.1 to 2% extract, about 0.001 to 1% extract, about 0.01 to 1% extract, or about 0.1 to 1% extract. In other embodiments, a specific weight of powder is added to the pharmaceutically acceptable carrier or other excipient. Accordingly, in some embodiments, the compositions of the present invention comprise on a weight/weight basis (weight of extract per total weight of the formulation), for example, from about 0.001 to 50% extract, about 0.01 to 50% extract, about 0.1 to 50% extract, about 0.001 to 10% extract, about 0.01 to 10% extract, about 0.1 to 10% extract, about 0.001 to 5% extract, about 0.01 to 5% extract, about 0.1 to 5% extract, about 0.001 to 4% extract, about 0.01 to 4% extract, about 0.1 to 4% extract, about 0.001 to 2% extract, about 0.01 to 2% extract, about 0.1 to 2% extract, about 0.001 to 1% extract, about 0.01 to 1% extract, or about 0.1 to 1% extract.

The present invention may be formulated as necessary with additives used commonly in the pharmaceutical sciences, such as surfactants, oils and fats, polyhydric alcohols, lower alcohols, thickening agents, UV absorbents, light scattering agents, preservatives, antioxidants, antibiotics, chelating agents, pH regulators, flavoring agents, pigments and water.

Examples of surfactants include polyoxyethylene (hereinafter abbreviated as POE-branched alkyl ethers such as POE-octyldodecyl alcohol and POE-2-decyltetradecyl alcohol, POE-alkyl ethers such as POE-oleyl alcohol ether and POE-cetyl alcohol ether, sorbitan esters such as sorbitan monooleate, sorbitan monoisostearate and sorbitan monolaurate, POE-sorbitan esters such as POE-sorbitan monooleate, POE-sorbitan monoisostearate and POE-sorbitan monolaurate, fatty acid esters of glycerol such as glyceryl monooleate, glyceryl monostearate and glyceryl monomyristate, POE-fatty acid esters of glycerol such as POE-glyceryl monooleate, POE-glyceryl monostearate and POE-glyceryl monomyristate, POE-dihydrocholesterol ester, POE-hardened castor oil, POE-hardened castor oil fatty acid esters such as POE-hardened castor oil isostearate, POE-alkylaryl ethers such as POE-octylphenol ether, glycerol esters such as glycerol monoisostearate and glycerol monomyristate, POE-glycerol ethers such as POE-glycerol monoisostearate and POE-glycerol monomyristate, polyglycerol fatty acid esters such as diglyceryl monostearate, decaglyceryl decastearate, decaglyceryl decaisostearate and diglyceryl diisostearate and other nonionic surfactants; potassium salts, sodium salts, diethanolamine salts, triethanolamine salts, amino acid salts and other salts of higher fatty acids such as myristic acid, stearic acid, palmitic acid, behenic acid, isostearic acid and oleic acid, the above alkali salts of ether carboxylic acids, salts of N-acylamino acids, N-acylsalconates, higher alkylsulfonates and other anionic surfactants; alkylamine salts, polyamine, aminoalcohol fatty acids, organic silicone resin, alkyl quaternary ammonium salts and other cationic surfactants; and lecithin, betaine derivatives and other amphoteric surfactants.

Examples of oils and fats include vegetable oils and fats such as castor-oil, olive oil, cacao oil, camellia oil, coconut oil, wood wax, jojoba oil, grape seed oil and avocado oil; animal oils and fats such as mink oil and egg yolk oil; waxes such as beeswax, whale wax, lanolin, carnauba wax and candelilla wax; hydrocarbons such as liquid paraffin, squalene, microcrystalline wax, ceresine wax, paraffin wax and vaseline; natural or synthetic fatty acids such as lauric acid, myristic acid, stearic acid, oleic acid, isostearic acid and behenic acid; natural or higher alcohols such as cetanol, stearyl alcohol, hexyldecanol, octyldecanol and lauryl alcohol; and esters such as isopropyl myristate, isopropyl palmitate, octyldodecyl myristate, octyldodecyl oleate and cholesterol oleate.

Examples of polyhydric alcohols include ethylene glycol, polyethylene glycol, propylene glycol, 1,3-butyrene glycol, 1,4-butyrene glycol, dipropylene glycol, glycerol, diglycerol, triglycerol, tetraglycerol and other polyglycerols, glucose, maltose, maltitose, sucrose, fructose, xylitose, sorbitol, maltotriose, threitol and erythritol.

Examples of thickening agents include naturally-occurring high molecular substances such as sodium alginate, xanthene gum, aluminum silicate, quince seed extract, gum tragacanth, starch, collagen and sodium hyaluronate; semi-synthetic high molecular substances such as methyl cellulose, hydroxyethyl cellulose, carboxymethyl cellulose, soluble starch and cationized cellulose; and synthetic high molecular substances such as carboxyvinyl polymer and polyvinyl alcohol.

Examples of UV absorbents include p-aminobenzoic acid, 2-ethoxyethyl p-methoxycinnamate, isopropyl p-methoxycinnamate, butylmethoxybenzoylmethane, glyceryl-mono-2-ethylhexanoyl-di-p-methoxybenzophenone, digalloyl trioleate, 2,2'-dihydroxy-4-methoxybenzophenone, ethyl-4-bishydroxypropylaminobenzoate, 2-ethylhexyl-2-cyano-3,3'-diphenyl acrylate, ethylhexyl p-methoxycinnamate, 2-ethylhexyl salicylate, glyceryl p-aminobenzoate, homomethyl salicylate, methyl o-aminobenzoate, 2-hydroxy-4-methoxybenzophenone, amyl p-dimethylaminobenzoate, 2-phenylbenzoimidazole-5-sulfonic acid and 2-hydroxy-4-methoxybenzophenone-5-sulfonic acid.

Examples of preservatives include benzoates, salicylates, sorbates, dehydroacetates, p-oxybenzoates, 2,4,4'-trichloro-2'-hydroxydiphenyl ether, 3,4,4'-trichlorocarbanilide, benzalkonium chloride, hinokitiol, resorcinol and ethanol.

Examples of antioxidants include tocopherol, ascorbic acid, butylhydroxyanisole, dibutylhydroxytoluene, nordihydroguaiaretic acid and propyl gallate.

Examples of chelating agents include sodium edetate and sodium citrate.

Examples of antibiotics include penicillin, neomycin, cephalothin, potassium permanganate, selenium sulfide, erythromycin, bacitracin, tethacyclin, chloramphenicol, vancomycin, nitrofurantoin, acrisorcin, chlorodontoin, and flucytosine.

Some of these additives function to enhance the efficacy of the composition by increasing the stability or percutaneous absorbability of the essential components of the present invention.

Also, any dosage form is acceptable, whether in solution, emulsion, powder dispersion, or others. Applicability is wide, including fundamental dosage forms such as lotions, emulsions, creams and gels.

In addition to those stated above, suitable vehicles, carriers and adjuvants include water, vaseline, petrolatum, mineral oil, vegetable oil, animal oil, organic and inorganic waxes, polymers such as xanthanes, gelatin, cellulose, collagen, starch, kaolin, carrageenan, gum arabic, synthetic polymers, alcohols, polyols, and the like. The carrier can also include sustained release carrier such as lypizomes, microsponges, microspheres, or microcapsules, aqueous base ointments, water in oil or oil in water emulsions, gels or the like.

### EXAMPLES

### Example 1

### Preparation of fish egg extracts

Fresh, unfertilized salmon (*Salmo salar*) eggs harvested from females in reproductive phase (late fall) are kept on ice, and the extract preferably made immediately. It is possible to freeze dry eggs in a cryoprotectant (e.g., 1.5 M 1,2-propanediol and 0.2 M sucrose) without disrupting the egg membrane. Freezing should be gradual (-1°C/min) to -80°C. Eggs should be thawed and kept on ice throughout the extract preparation procedure.

Eggs are washed twice in HBSS or seawater with protease inhibitors (10ug/ml). The washing solution is removed and the eggs are lysed and homogenized in a pre-chilled Dounce glass-glass homogenizator. The lysate is transferred to Beckman Ultra Clear polyallomer centrifuge tubes (5 ml) while avoiding transfer of egg shells, and centrifugated for 15 min at 15.000g at 4°C in a Beckman ultracentrifuge using SW55T1 rotor. Three fractions are thereby obtained; lipid top fraction, cytoplasmic middle fraction, and a bottom fraction containing eggshells and nucleic debris. The cytoplasmic middle fraction is the collected extract. This extract is expected to contain most cytosolic organelles including mitochondria, lysosomes and peroxisomes, should be clear and viscous, and have an orange tint. Protease inhibitors (10ug/ml stock) are added and extracts are kept at -80°C.

Further fractionation of the cytoplasmic extract is possible. Centrifugation at 100,000g at 4°C for 60 minutes yields 2-3 fractions, where the top/middle cytoplasmic fraction contains the cytosol with endoplasmic reticulum, SV and microsomes. The extract pH is measured by litmus paper, protein concentration measured by Bradford assay, and osmolarity measured by osmometer.

Mid-blastula Zebra fish embryos are collected, liquid removed and frozen to -20°C. To prepare the extract, embryos are thawed on ice, lysed and homogenized by Dounce glass-glass homogenizator in a small amount of either HBSS or seawater (preferably less than 50% liquid v/v). The lysate is filtered through a sterile linen cloth and centrifugated at 5,000g at 4°C for 20 minutes in a SX4250 rotor using a Beckman X-22R centrifuge. The cytoplasmic extract (supernatant) is collected and protease inhibitors (10ug/ml) are added. The extract may be Millipore filtered (0.22 um MilliQ sterile filter). The extracts are kept at -80°C. The extract pH is measured by litmus paper, protein concentration measured by Bradford assay, and osmolarity measured by osmometer.

This general procedure is useful for the preparation of extracts from sea urchin, shrimp, fish eggs/roe or frog eggs. Briefly, roe collected from gravid female fish soon after they liberated their eggs in a spawning program (hCG hormone injected (1 ml/kg) at 6 to 8 hours before egg liberation, usually at dawn (2 - 4 am), or from gravid frogs. Roe/eggs are freeze dried or frozen at -20°C or used fresh. Roe is collected from different kinds of fish. For sea-urchin, 0.5 M KCl is injected around the mouth to evoke shedding of eggs. The extract is prepared from eggs/roe by crushing (cell cracker or dounce-homogenization) or centrifugation at different speeds to separate cytoplasm with all content, with/without eggshells (zona pellucida), with/without nucleus/cytosol, with/without organelles, with/without lipids. Further fractionation can be conducted to isolate one or more of mRNA, proteins, small peptides, carbohydrates and lipids. Major components of fatty acids in the roe are oleic acid, linoleic acid, and omega-3 fatty acids.

Upon application of the above protocol for salmon egg extracts, the salmon egg extracts had a surprisingly high protein concentration varying from 100 - 380 mg/ml, pH between 6.4 - 6.8, and an osmolarity of approximately 350 mOsm. The extracts were clear and viscous and non-filterable (by 0.45 um MilliQ filter). The protein in the extract precipitated easily upon addition of water or hydrous solutions with low buffering capacity due to the high protein content and low pH. Extracts could be neutralized to pH 7.0 by addition of alkaline (1-3 ul 1M NaOH/ml extract), whereupon dilution in water and hydrous solutions was possible. Zebra-fish extracts had a protein concentration varying from 23 - 26 mg/ml, pH between 6.4 - 6.8, and an osmolarity between 80 - 150 mOsm. The extracts were clear and non-viscous, filterable and diluted readily in water at all dilutions.

Physical properties of various fish egg extracts, including RNA, DNA and protein content, made by these methods were were further assessed using the Qube-iT fluorimeter from InVitrogen.

Homogenates of salmon eggs (non-centrifugated) contain 3-4 mg/ml RNA. After centrifugation to 9-15,000g, RNA content was reduced to 2-3 mg/ml. This is probably due to RNA being centrifugated down or degraded. Interestingly, trout egg homogenates (non-centrifugated) contain 2-3 mg/ml RNA, but after centrifugation to 9-15,000g, the concentration of RNA sis increased to 3-5 mg/ml. Extracts made from trout eggs are less viscous than extracts made from salmon eggs, and may keep RNA better in water phase suspension during centrifugation.

### Homogenates of salmon eggs (non-centrifugated) contain 60-200 ug/ml DNA

After centrifugation to 9-15,000g, DNA content was reduced to 40-51 ug/ml. This is probably due to DNA being centrifugated down. Interestingly, homogenates of trout eggs (non-centrifugated) contain more DNA than salmon egg extracts: 130-530 ug/ml DNA. After centrifugation to 9-15,000g, DNA content is reduced to 70-125 ug/ml, but is still higher than comparable salmon egg extracts. Extracts made from trout eggs are less viscous than extracts made from salmon eggs, and may keep DNA in better water phase suspension during centrifugation.

Homogenates of salmon eggs (non-centrifugated) contain 180-260 mg/ml protein. After centrifugation to 9-15,000g, protein content was unchanged or increased slightly to 200-260 mg/ml. Homogenates of trout eggs (non-centrifugated) contain 250-300 mg/ml protein, and after centrifugation to 9-15,000g, protein content is roughly the same (250-270 mg/ml). The protein fraction of the egg cytosol is not expected to be spun down at the g-forces applied, and may be expected to be similar to the raw protein content of the egg cytosol.

Previous measurements of protein contents in extracts using a Nano-drop spectrophometer showed a range of 150-250 mg/ml. This may be due to an upper detection limit around 250 mg/ml in the Nano-drop. It is probable that the slightly higher fluorometer measurements presented here are more accurate.

**Table 1. Summary of measurements RNA, DNA and protein content in extracts**

| Source of eggs | Centrifugation speed | LEX/corresp to LEX | mg/ml RNA | µg/ml DNA | mg/ml protein |
|---|---|---|---|---|---|
| Salmon | Homogenate, no centrifugation | LEX20 | 3.51 | 66.8 | 256 |
| Salmon | 15000 xg | LEX20 | 2.34 | 44 | 252 |
| Salmon | Homogenate, no centrifugation | LEX24 | 3.42 | 192.4 | 180 |
| Salmon | 12000 xg | LEX24 | 2.93 | 50.8 | 208 |
| Trout | Homogenate, no centrifugation | LEX28 | 2.67 | 131.6 | 249 |
| Trout | 15000 xg | LEX28 | 3.51 | 73.2 | 249 |
| Trout | Homogenate, no centrifugation | LEX25 | 2.53 | 528 | 296 |
| Trout | 15000 xg | LEX25 | 3.70 | 72.8 | 262 |
| Trout | 15000 xg | LEX25 | 3.63 | 99.2 | 210 |
| Trout | 12000 xg | LEX31 | 4.59 | 87.2 | 270 |
| Trout | 12000 xg | LEX32 | 4.68 | 124.8 | - |
| Trout | 12000 xg | LEX33 | 4.67 | 94.4 | 252 |

The lipid content of extracts were measured by ALS (Germany), and was found to be in the narrow range of 3.7 - 4.5 g/100g in all extracts from salmon or trout roe prepared at centrifugations spanning from 1,700g to 15,000g. The lower g-force centrifugations appear to require spinning at room temperature to give equal lipid fractionation to higher g-forces at 4 degrees centigrade. At lower centrifugal forces than 1,700g also applicable to produce an extract the lipid content may be higher (4-7%). The extract may contain the following lipids (right column), and the lipid fraction removed from the extract during production may include the following lipids (left column)

**Table 2. Lipid content can vary beetween batch production and specific lipids can include:**

| Report by ALS Scandinavia | | Removed from extract | % of remaining lipids in extract |
|---|---|---|---|
| **ELEMENT** | SAMPLE | LIPID lipid fract. | LEX 42 |
| Fatty acids, saturated | g/100g | 22,7 | 1,6 |
| Fatty acids, monounsaturated | g/100g | 36,6 | 2,8 |
| Fatty acids, polyunsaturated | g/100g | 44,5 | 0,8 |
| C4:0 Butyric acid | g/100g | <0.10 | <0.10 |
| C6:0 Caproic acid | g/100g | <0.10 | <0.10 |
| C8:0 Caprylic acid | g/100g | <0.10 | 0,25 |
| C10:0 Capric acid | g/100g | <0.10 | 0,62 |
| C11:0 Undecanoic acid | g/100g | <0.10 | <0.10 |
| C12:0 Lauric acid | g/100g | <0.10 | <0.10 |
| C13:0 Tridecanoic acid | g/100g | <0.10 | <0.10 |
| C14:0 Myristic acid | g/100g | 3 | 4,2 |
| C14:1 Myristoleic acid | g/100g | 0,11 | 0,15 |
| C15:0 Pentadecanoic acid | g/100g | 0,3 | 0,34 |
| C15:1 cis10-Pentadecanoic acid | g/100g | <0.10 | 0,12 |
| C16:0 Palmitic acid | g/100g | 11 | 14,3 |
| C16:1 Palmitoleic acid | g/100g | 7,3 | 9,6 |
| C17:0 Heptadecanoic acid | g/100g | 0,24 | 0,21 |
| C17:1 Heptadecenoic acid | g/100g | <0.10 | <0.10 |
| C18:0 Stearic acid | g/100g | 3,6 | 5,4 |
| C18:1 Oleic acid | g/100g | 22,9 | 33,8 |
| C18:2 Linoleic acid (omega6) | g/100g | 6,2 | 5 |
| C18:3 Linolenic acid(omega6) | g/100g | 0,92 | 0,47 |
| C18:3 a-Linolenic acid(omega3) | g/100g | 2,4 | <0.10 |
| C18:4 Stearidonic acid (ome3) | g/100g | 0,57 | 0,23 |
| C20:0 Arachidic acid | g/100g | 0,85 | 0,43 |
| C20:1 Eicosenoic acid | g/100g | 0,83 | 1,1 |
| C20:2 Eicosadienoic acid (om6) | g/100g | 0,49 | 0,34 |
| C20:3Eicosatrienoic ac(omega6) | g/100g | 0,3 | 0,14 |
| C20:4 Arachidonic acid(omega6) | g/100g | 1,6 | 0,78 |
| C20:5Eicosapentaenoic ac(ome3) | g/100g | 9,4 | 2,8 |
| C21:0 Heneicosanoic acid | g/100g | <0.10 | <0.10 |
| C22:0 Behenic acid | g/100g | 3,1 | 1,3 |
| C22:1 Erucic acid | g/100g | <0.10 | <0.10 |
| C22:2 Docosadienoic ac (ome6) | g/100g | <0.10 | <0.10 |
| C22:5 Docosapentaenoic ac(ome3 | g/100g | 6,8 | 2,3 |
| C22:6 Docosahexaenoic ac(ome3) | g/100g | 13,5 | <0.10 |
| C24:0 Lignoceric acid | g/100g | <0.10 | <0.10 |
| C24:1 Nervonic acid | g/100g | <0.10 | 2,6 |
| Vanninnhold (water) | g/100g | | 65,9 |

### Example 2

### Production of extracts

As described in Example 1, the lipid content of the extract surprisingly is unchanged at centrifugation speeds varying from 1,700g to 15,000g (see above), while other parameters such as RNA, DNA and protein content is altered with the increase of g-force during centrifugation. An extract may also be produced by lower centrifugal forces, down to 400g, whereupon the content of especially lipids may be higher.

An extra step of washing the eggs for 10 minutes with buffodine (1:100 in 0.9% NaCl) before preparation of homogenate is beneficial. This washing step appears to reduce the bacterial content significantly. For safety reasons, all LEX batches packaged in final containers are mildly pasteurized (incubated) by heating to 56 °C for 20 minutes. This pasteurization sterilizes the extract completely, with 0 bacteria found in extracts plated on bacteria dishes incubated for 3 days at room temperature, 4 degrees centigrade or 30 degrees centigrade. 1 colony/100ul LEX plated on agar dish incubated at room temperature is the maximum observed. This is 100x below safety limits for drinking water (100 bacteria/ml). A single colony seldom observed probably comes from the air during the plating of LEX, and is comparable to bacterial growth of negative control (plate only).

The stability of LEX and collagen secretion effect is retained after LEX is heated to 56 C for 20 minutes. When applied to human fibroblasts *in vitro* at 0.5% concentration in cell media for 8 days (media changed daily), the effect on collagen secretion (as measured as efflux of collagen from cells to cell medium and compared to untreated control cells), was comparable to cells treated with unheated extract which had been kept at -80C after preparation. A 200-400% increase compared to controls was observed for both heated and unheated LEX.

Heat treated LEX (HEX) is produced produced from salmon egg extract (LEX) prepared as described above. For HTX, the Salmon egg extract was tenfold diluted (1:10) in sterile 1xPBS buffer; 0.5ml aliquots were prepared. All samples were heated at 95°C for five minutes, cooled down and then centrifuged at 12,100 x g for 2 min. The supernatant were collected, and the samples were labeled and stored at -80 °C until usage.

### Example 3

This example describes the use of fish egg extracts (e.g., LEX or HEX produced as decribed above from salmon eggs and trout eggs) to modulate the synthesis and/or release of cell mediators by macrophages. Human monocytes are obtained from healthy blood donors by an established 2-step isolation procedure using the separation gradient percoll^{™} (Pharmacia, Sweden). Pertoft H, Johnsson A, Wärmegård B, Seljelid R. Separation of human monocytes on density gradients of Percoll. J Immunol Methods 1980;33:221-9. Buffy coat is layered over 1.076 g/ml percoll^{™} in conical centrifugation tubes and centrifuged at 800g for 30 min at room temperature. The layer containing mononuclear cells is then aspirated, washed twice with cold Hanks' balanced salt solution (HBSS, without Ca²⁺ and Mg²⁺), layered on top of 1.064 g/ml Percoll^{™} and centrifuged at 800g for 60 min at 4 °C. The layer of monocytes is harvested and washed three times in HBSS. The cell concentration is estimated by cells being permeabilised and stabilized before being stained with propidium iodide in the NucleoCassette^{™} and counted by the NucleoCounter^{™} (Chemometec, Allerød, Denmark), according to the manufacturer's protocol. The isolated monocytes are then resuspended to a concentration of 5X10⁵ cells/ml in Roswell Park Memorial Institute (RPMI) 1640 culture medium (Invitrogen, Paisley, UK), supplemented with 5% fetal bovine serum, 100 U/ml penicillin, 100 µg/ml streptomycin and 2.5 µg/ml Fungizone^{™} (Invitrogen). The viability of the cells is checked by trypan blue dye exclusion. After monocyte isolation, 5X10⁵ cells per well are added to 24-well plates (Falcon^{™}, BD Biosciences, Bedford, MA, USA) and cultured for 24 h at 37 °C with 5% CO₂ and 95% humidity. Thereafter, supernatants, including nonadherent cells, are removed resulting in the increased purity of the monocyte population by the removal of potential remaining lymphocytes (the adherent monocytes are referred to here as monocyte-derived macrophages or only macrophages). This procedure is repeated for six donors.

Next, fresh medium containing LPS, and 1%, 2% or 5% LEX or HEX, is added to the wells in triplicate. The plates are incubated for 6, 24 and 72 h at 37 °C with 5% CO₂ and 95% humidity. Cells are counted by the NucleoCounter^{™} system during cultivation and cell numbers are found to be constant in all wells during the entire experiment. No giant cells are detected, after 6-72 h. Thereafter, the supernatants are collected, centrifuged at 400g for 5 min at 5 °C and stored at -80 °C until analysis. Samples without added LEX/HEX are used as controls. Samples with IL-4 (interleukin-4, 20 ng/ml) treatment are prepared specifically for an AMAC-1 elisa assay, where IL-4 is used as a positive control. Controls showing the monocyte inflammatory response with neither LPS nor added LEX/HEX are also prepared. Cell viability is evaluated by measuring the levels of lactate dehydrogenase in culture medium colorimetrically. High viability is observed in all samples.

Conditioned medium from the monocyte cultures is analysed for cytokine concentrations using the human cytokine 25-plex bead immunoassay (Biosource^{™}; Invitrogen). The measurements are performed according to the manufacturer's instructions. Briefly, samples are clarified by centrifugation at 17,000g at 4 °C for 10 min and are added to a filter bottom microplate containing beads conjugated to cytokine-specific antibodies. The microplate is incubated for 2 h at room temperature on an orbital shaker. Thereafter, analyte-specific biotinylated detector antibodies are added to each well, followed by1 h of incubation. Streptavidin conjugated to R-Phycoerythrin (RPE) is pipetted into each well and the microplate is incubated for 30 min. The beads are resuspended in wash solution and the microplate is analysed using a Luminex 100^{™} cytometer (Applied Cytometry System, Sheffield, UK). The concentrations of the cytokines are determined by monitoring the spectral properties of the beads while simultaneously quantifying associated RPE fluorescence. Monocyte-conditioned media is also analyzed for the following cytokines and growth factors using commercial ELISA kits: Alternative Macrophage Activation-Associated CC chemokine-1 (AMAC-1; Human PARC/CCL18 DuoSet ELISA Development Kit, R&D Systems), VEGF and insulin-like growth factor-1 (IGF-1; R&D Systems). The optical density is measured with a plate reader (Spectramax, Molecular Devices, UK) and translated to cytokine levels using Softpro Software (Molecular Devices, UK).

The human cytokine 25-plex bead immunoassay (Biosource^{™}; Invitrogen) enables the detection of human eotaxin, granulocyte macrophage colony-stimulating factor (GM-CSF), interferon-alpha (IFN-α), IFN-γ, interleukin-1beta (il-1β), interleukin-1 receptor antagonist (IL-1RA), IL-2, interleukin-2 receptor (IL-2R), IL-4, IL-5, IL-6, IL-7, IL-8, IL-10, IL-12p40/p70, IL-13, IL-15, IL-17, immune protein-10 (IP-10), monocyte chemotactic protein-1 (MCP-1), monokine induced by gamma-interferon (MIG), macrophage inflammatory protein-1alpha (MIP-1α), MIP-1β, RANTES and tumor necrosis factor-alpha (TNF-α).

The following results are obtained. The group of macrophages treated with 1%, 2% or 5% LEX or HEX from salmon or trout eggs exhbit an increase in expression and release the following cell mediators: IL-1 RA, IL-6, IL-10 and VEGF as compared to controls; a decrease in expression and release by macrophages of the following cell mediators: IL-12, TNF-α, MCP-1, and IL-8 as compared to controls; and an increase in expression by macrophages of AMAC-1 as compared to controls.

### Example 4

All animals used in this study are housed in a certified animal care facility. All procedures are approved by an ethical review committee. *Leprdb*/*db* and *Leprdb*/+ mice are purchased from the Jackson Laboratory (Bar Harbor, ME) and Harlan (Oxfordshire, UK). Heterozygous animals are crossed to the C57BL/6-Tg(CAG-EGFP)1Osb/J line, which ubiquitously express enhanced GFP (Jackson Labs), and subsequently backcrossed to produce *Leprdb*/*db* animals carrying the eGFP transgene. All animals are used between 8 and 16 weeks of age, and are age and sex-matched to controls.

Diabetic and non-diabetic control animals are anaesthetized and the dorsum shaved and sterilized with antiseptic wipes. Full thickness wounds of 8 mm diameter are excised, including the panniculus carnosus layer. Animals received buprenorphine at the time of surgery and are housed in separate cages until tissue is harvested at the described time points by removing the entire wound area, including a 2 mm perimeter.

Wounds are harvested from sacrificed animals, cut in half and fixed in formalin overnight, then embedded in paraffin. Wounds are then cut into 5-µm sections using a Microm HM 330 microtome and stained using haemotoxylin and eosin to locate matching section morphology for further analysis. Sections are then dewaxed and rehydrated, followed by sodium citrate buffer antigen retrieval. Sections are incubated with the following antibodies, in various combinations depending on the experiment, diluted in PBT (PBS + 0.1% Tween-20): rat anti-CD45 (1:50, Invitrogen/Life Technologies, clone 30- F11, MCD4500), goat anti-Arg1 (1:200, Santa Cruz Biotechnology, sc-18354), rabbit anti-Nos2 (1:100, Santa Cruz Biotechnology, sc-651), rabbit anti-GFP (1:1000, Abcam, ab6556), followed by the following secondary antibodies as appropriate: donkey anti-rat Alexa Fluor 488 (1:500, Invitrogen/Life Technologies, A-21208), donkey anti-goat Cy5 (1:500, Abcam, ab6566), donkey anti-rabbit Alexa Fluor 555 (1:500, Invitrogen/Life Technologies, A-31572).

Sections are then mounted in Prolong Gold with DAPI (Invitrogen/Life Technologies, P36930) and images captured sequentially on an inverted Olympus FV1000 confocal microscope. Three non-db, three db animals, three db animals treated with 2% or 5% LEX or HEX, and three non-db animals treated with 2% or 5% LEX or HEX are used for each time point. Time points are day 1, day 4 and day 7. Four fields of view from each wound are taken from both the peri-wound dermis and the granulation tissue in consistent locations from wound to wound. Images are then analysed to determine the number of CD45+ Nos2+ Arg1+, CD45+ Nos2+ Arg1- and CD45+ Nos2- Arg1+ cells in each sample using Photoshop to determine the marker overlap. The percentage of each (double positive or triple positive) is calculated and is reported as the mean ± s.e.m. of all images for each category (n=12 for each).

The results show that db animals with wounds treated with 2% or 5% LEX or HEX have an increased ratio of alternatively activated macrophages: classically activated macrophages at the site of the wounds at days 4 and 7 as compared to control antimals (i.e., untreated db animals).

### Example 5

This example shows that salmon roe extract reduces expression of inflammatory markers following stimulation in *in vitro* models.

**Materials and Methods.** Human foreskin fibroblasts were purchased from ATCC (Cat nr ATCC^{®} CRL-2522) and cultured as previously described. Where relevant human foreskin fibroblasts were grown to confluence in a T75 flask. Cells were then incubated for 24 hrs in the presence of 5% HTX or in the absence of any treatment. Fibroblasts were stimulated with 0.1 to 10 nM TNFα for one hour. Post treatment relevant markers were quantified. The relative reduction of expression is represented as fold decrease below the control sample.

Human macrophages were isolated using the method of Menc et al. 2004. Macrophages were suspended at 5 x 105 cells/ml. Untreated confluent monolayers, monolayers treated with 5% HTX, or monolayers treated with IL-4 treatment were stimulated with 100 ng/ml LPS and 10-10,000 U/ml INF-γ for up to 72 hours. ELISA was employed to measure IL-1β in the relevant cell lines.

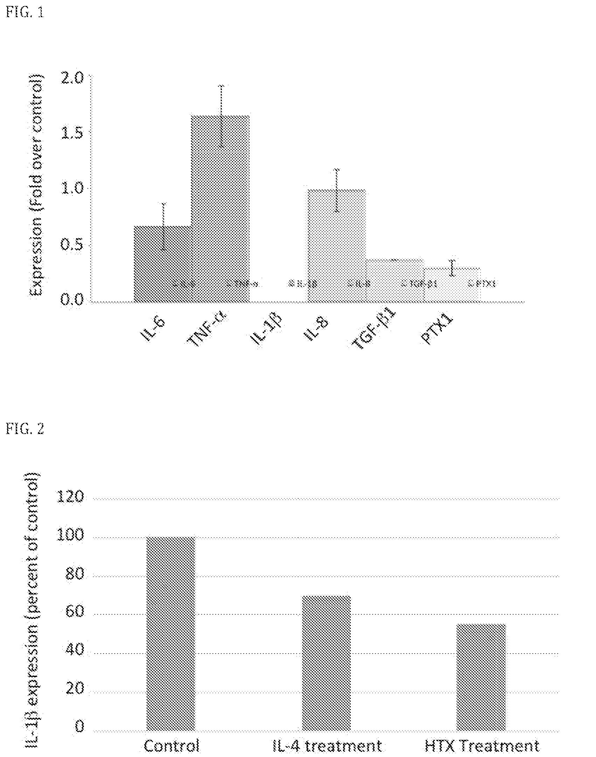

**Conclusions.** Figure 1 shows reduction of selected inflammatory markers in human fibroblasts pretreated with salmon roe extract (0.5 % w/v) vs. control (no pre-treatment). In all treatment groups, fibroblasts were stimulated with TNF-α (0.1 to 10 nM) to induce markers. Fold reduction of: IL-6 (65%) , TNF-α (150%) , IL-1 β (0%), IL-8 (100%), TGF-1β (35%), PTX1 (CRP), (35%).

Figure 2 shows reduction of IL-1β in human macrophages pretreated with salmon roe extract (0.5 % w/v) vs. control (no pre-treatment). In all treatment groups, macrophages were pre-stimulated with LPS/IFN-γ for 72 h to induce markers. Fold reduction of: IL-1 β (55%), IL-4 (65%).

## Claims

1. Egg cellular extract for use in promoting, accelerating, or improving healing of chronic wound in a diabetic subject, wherein said egg cellular extract is fish egg cellular extract.

2. Egg cellular extract for use according to claim 1, wherein said chronic wound is a diabetic ulcer.

3. Egg cellular extract for use according to claim 1 or 2, wherein the use increases the ratio of alternatively activated macrophages: classically activated macrophages at the site of the wound upon application of said egg cellular extract.

4. Egg cellular extract for use according to any of claims 1 to 3, wherein the use increases the expression and/or release by macrophages of one or more cell mediators selected from the group consisting of IL-1 RA, IL-6, IL-10 and VEGF.

5. Egg cellular extract for use according to any of claims 1 to 4, wherein the use decreases the expression and/or release by macrophages of one or more cell mediators selected from the group consisting of IL-12, TNF-α, MCP-1, and IL-8.

6. Egg cellular extract for use according to any of claims 1 to 5, wherein the use increases expression of AMAC-1 by macrophages .

7. Egg cellular extract for use according to any one of claims 1 to 6, wherein said fish egg cellular extract is from an unfertilized egg.

8. Egg cellular extract for use according to any of claims 1 to 7, wherein said cellular extract is obtained by heat treating the cellular extractby heating the extract to greater than 80C, 90C, 95C or 100C.

9. Egg cellular extract for use according to claim 8, wherein said heat treatment is from about 1 minute to about 30 minutes.

10. Egg cellular extract for use according to any of claims 1 to 9, wherein said egg cellular extract is provided in a cream, gel, emulsion, ointment, spray, powder or lotion.

11. Egg cellular extract for use according to any of claims 1 to 10, wherein said egg cellular extract is a cytoplasmic extract.

12. Egg cellular extract for use according to any of claims 1 to 11, wherein the use produces the effect as defined in claims 3-6 at the site of a wound in a subject.

## Patentansprüche

1. Eizellextrakt zur Verwendung bei der Förderung, Beschleunigung oder Verbesserung der Heilung einer chronischen Wunde bei einem Diabetiker, wobei es sich bei dem Eizellextrakt um Fischeizellextrakt handelt.

2. Eizellextrakt zur Verwendung nach Anspruch 1, wobei es sich bei der chronischen Wunde um ein diabetisches Ulkus handelt.

3. Eizellextrakt zur Verwendung nach Anspruch 1 oder 2, wobei durch die Verwendung an der Stelle der Wunde bei einer Applikation des Eizellextrakts das Verhältnis alternativ aktivierte Makrophagen:klassisch aktivierte Makrophagen erhöht wird.

4. Eizellextrakt zur Verwendung nach einem der Ansprüche 1 bis 3, wobei durch die Verwendung die Expression und/oder Ausschüttung eines oder mehrerer Zellmediatoren, die ausgewählt sind aus der Gruppe bestehend aus IL-1 RA, IL-6, IL-10 und VEGF, durch Makrophagen erhöht werden.

5. Eizellextrakt zur Verwendung nach einem der Ansprüche 1 bis 4, wobei durch die Verwendung die Expression und/oder Ausschüttung eines oder mehrerer Zellmediatoren, die ausgewählt sind aus der Gruppe bestehend aus IL-12, TNF-α, MCP-1 und IL-8, durch Makrophagen verringert werden.

6. Eizellextrakt zur Verwendung nach einem der Ansprüche 1 bis 5, wobei durch die Verwendung die Expression von AMAC-1 durch Makrophagen erhöht wird.

7. Eizellextrakt zur Verwendung nach einem der Ansprüche 1 bis 6, wobei der Fischeizellextrakt von einem unbefruchteten Ei stammt.

8. Eizellextrakt zur Verwendung nach einem der Ansprüche 1 bis 7, wobei der Zellextrakt durch eine Wärmebehandlung des Zellextrakts mittels Erwärmen des Extrakts auf mehr als 80 °C, 90 °C, 95 °C oder 100 °C erhalten wird.

9. Eizellextrakt zur Verwendung nach Anspruch 8, wobei die Wärmebehandlung von etwa 1 Minute bis etwa 30 Minuten dauert.

10. Eizellextrakt zur Verwendung nach einem der Ansprüche 1 bis 9, wobei der Eizellextrakt in einer Creme, einem Gel, einer Emulsion, einem Zäpfchen, Spray, Pulver oder einer Lotion bereitgestellt wird.

11. Eizellextrakt zur Verwendung nach einem der Ansprüche 1 bis 10, wobei es sich bei dem Eizellextrakt um einen Zytoplasmaextrakt handelt.

12. Eizellextrakt zur Verwendung nach einem der Ansprüche 1 bis 11, wobei durch die Verwendung an der Stelle einer Wunde in einem Individuum die in den Ansprüchen 3-6 definierte Wirkung erzeugt wird.

## Revendications

1. Extrait cellulaire d'œuf pour une utilisation dans la promotion, l'accélération ou l'amélioration de la cicatrisation d'une plaie chronique chez un sujet diabétique, dans lequel ledit extrait cellulaire d'œuf est un extrait cellulaire d'œuf de poisson.

2. Extrait cellulaire d'œuf pour une utilisation selon la revendication 1, dans lequel ladite plaie chronique est un ulcère diabétique.

3. Extrait cellulaire d'œuf pour une utilisation selon la revendication 1 ou 2, dans lequel l'utilisation augmente le rapport entre macrophages activés en alternance et macrophages activés de manière classique sur le site de la plaie lors de l'application dudit extrait cellulaire d'œuf.

4. Extrait cellulaire d'œuf pour une utilisation selon l'une quelconque des revendications 1 à 3, dans lequel l'utilisation augmente l'expression et/ou la libération, par les macrophages, d'un ou plusieurs médiateurs cellulaires choisis dans le groupe constitué par IL-1 RA, IL-6, IL-10 et VEGF.

5. Extrait cellulaire d'œuf pour une utilisation selon l'une quelconque des revendications 1 à 4, dans lequel l'utilisation diminue l'expression et/ou la libération, par les macrophages, d'un ou plusieurs médiateurs cellulaires choisis dans le groupe constitué par IL-12, TNF-α, MCP-1 et IL-8.

6. Extrait cellulaire d'œuf pour une utilisation selon l'une quelconque des revendications 1 à 5, dans lequel l'utilisation augmente l'expression d'AMAC-1 par les macrophages.

7. Extrait cellulaire d'œuf pour une utilisation selon l'une quelconque des revendications 1 à 6, dans lequel ledit extrait cellulaire d'œuf de poisson est issu d'un œuf non fécondé.

8. Extrait cellulaire d'œuf pour une utilisation selon l'une quelconque des revendications 1 à 7, dans lequel ledit extrait cellulaire est obtenu par traitement thermique de l'extrait cellulaire, en chauffant l'extrait à une température supérieure à 80°C, 90°C, 95°C ou 100°C.

9. Extrait cellulaire d'œuf pour une utilisation selon la revendication 8, dans lequel le traitement thermique a une durée comprise entre environ 1 minute et environ 30 minutes.

10. Extrait cellulaire d'œuf pour une utilisation selon l'une quelconque des revendications 1 à 9, dans lequel ledit extrait cellulaire d'œuf se présente sous forme de crème, de gel, d'émulsion, de pommade, de spray, de poudre ou de lotion.

11. Extrait cellulaire d'œuf pour une utilisation selon l'une quelconque des revendications 1 à 10, dans lequel ledit extrait cellulaire d'œuf est un extrait cytoplasmique.

12. Extrait cellulaire d'œuf pour une utilisation selon l'une quelconque des revendications 1 à 11, dans lequel l'utilisation produit l'effet tel que défini dans les revendications 3 à 6 sur le site d'une plaie chez un sujet.
